# EUROPÄISCHE PATENTSCHRIFT

(11) **EP 3 229 768 B1**
(45) Veröffentlichungstag und Bekanntmachung des Hinweises auf die Patenterteilung: **29.07.2020**
(21) Anmeldenummer: 15804131.9
(22) Anmeldetag: 03.12.2015
(51) Int. Cl.: A61K 8/44, A61Q 11/00

(54) **MUND- UND ZAHNPFLEGE- UND -REINIGUNGSMITTEL MIT ARGININDERIVAT ZUR VERBESSERTEN PLAQUEENTFERNUNG**
ORAL AND DENTAL HYGIENE AND CLEANING PRODUCTS WITH AN ARGININE DERIVATIVE FOR IMPROVED PLAQUE REMOVAL
PRODUITS DE SOIN ET D'HYGIÈNE BUCCO-DENTAIRE COMPRENANT UN DÉRIVÉ D'ARGININE POUR UNE MEILLEURE ÉLIMINATION DE LA PLAQUE

(30) Priorität: 10.12.2014 DE 102014225427
(43) Veröffentlichungstag der Anmeldung: 18.10.2017
(73) Patentinhaber: Henkel AG & Co. KGaA, 40589 Düsseldorf (DE)
(72) Erfinder: WELSS, Thomas, 40591 Düsseldorf (DE); DUSCHEK, Nicole, 40595 Düsseldorf (DE); MIEHLICH, Kristin, 42119 Wuppertal (DE)
(86) Internationale Anmeldenummer: PCT/EP2015/078451
(87) Internationale Veröffentlichungsnummer: WO 2016/091702

(56) Entgegenhaltungen:
- EP-A1- 1 584 320
- EP-A1- 2 377 848
- WO-A1-2012/057739
- US-A- 4 477 429
- US-A1- 2004 258 630

## Beschreibung

Die Erfindung betrifft Mund- und Zahnpflege- und -reinigungsmittel mit einem Arginin-Derivat zur schonenden und effektiven Reinigung der Zähne.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Ein ursächlicher Zusammenhang zwischen dem Vorhandensein von Zahnbelag (Plaque) und insbesondere von Zahnstein und verschiedenen Erkrankungen des Zahnfleischs (Gingiva) wie Karies, Gingivitis, Halitosis oder Parodontitis ist allgemein anerkannt.

Als Basis für eine geeignete Prophylaxe gilt das zwei- oder mehrmals am Tag durchzuführende Zähneputzen, um eine unerwünschte Belagbildung auf den Zahnoberflächen und insgesamt einen bakteriellen Befall des Mundraums zumindest einzudämmen.

Allerdings lässt sich bereits wenige Minuten nach dem Zähneputzen die Neubildung der sogenannten Pellikelschicht auf der Zahnoberfläche nachweisen. Dabei handelt es sich um einen Niederschlag aus Proteinen aus dem Speichel. Die Pellikelschicht ist für die Zahngesundheit an sich wenig bedenklich. Allerdings siedeln sich im Folgenden auf der Pellikelschicht weitere Mikroorganismen aus der Speichelflora an, was zu einem Dickenwachstum des entstandenen Biofilms führt. Hierbei können sich auch schädliche Mikroorganismen ansiedeln. Dabei kann eine Matrix aus extrazellulären polymeren Substanzen (EPS) entstehen, welche in der Lage ist, verschiedenste Bakterienspezies auf der Zahnoberfläche einzubetten. Die entstehende Zellschicht wird als Zahnbelag (Plaque) bezeichnet und fördert das Auftreten von Karies. Die in der Plaque enthaltenen Polysaccharide bilden neben niedermolekularen Zuckern eine Nahrungsquelle für die eingebetteten Bakterien. Die Bakterien bauen die Polysaccharide allmählich zu sauren Abbauprodukten wie Brenztraubensäure und Milchsäure ab. Die daraus resultierende pH-Absenkung bewirkt den als Karies bekannten Abbau des Zahnschmelzes.

Aus der Plaque kann durch Aufnahme und Einlagerung anorganischer Stoffe (Mineralstoffe) aus dem Speichel Zahnstein entstehen. Dieser ist in der Regel nicht mehr durch die Zahnbürste entfernbar. Der Zahnstein fördert das Auftreten der oben angeführten Erkrankungen des Zahnfleischs.

Aus der EP 2591766 A2 ist ein Mund- und Zahnpflege- und -reinigungsmittel mit einem ausgewählten Polyamin bekannt, welches in der Lage ist, Zahnbelag zu reduzieren.

Es besteht aber nach wie vor das Bedürfnis, Zahnbelag zu reduzieren, die daraus resultierenden Erkrankungen zu bekämpfen und Wirkstoffe dafür bereitzustellen. Es bestand weiter die Aufgabe, Mund- und Zahnpflege- und -reinigungsmittel entsprechend weiterzuentwickeln.

In nicht vorhersehbarer Weise wurde nun gefunden, dass die zuvor beschriebenen Aufgaben durch den Einsatz von Polyol-Derivaten von Arginin gelöst werden kann.

Der Einsatz von Arginin in Mund- und Zahnpflegemitteln ist an sich bekannt. So beschreibt die US 3,413,326 den Einsatz von HF-Additionsprodukten an Arginin als Anti-Karies-Verbindungen in Zahnpasten. Aus der WO 2012/57739 A1 sind Zahnpasten mit einer Kombination aus Arginin und Calciumcarbonat bekannt, die insbesondere zum Reinigen und Pflegen von überempfindlichen Zähnen geeignet sind.

Die Mund- und Zahnpflege- und -reinigungsmittel, umfassend Polyol-Derivate von Arginin, zeigen eine überraschend gute Wirkung bei der Inhibierung, Reduktion und Auflösung (Eliminierung) von Biofilmen auf Zahnoberflächen. Dabei wird die Pellikelschichtneubildung dahingehend modifiziert, dass die Anhaftung von Bakterien auf die gereinigten Zahnoberflächen erschwert wird. Außerdem werden gegebenenfalls vorhandene extrazelluläre polymere Strukturen (EPS) aufgelöst. Ebenso wird die pH-Absenkung an der Zahnoberfläche verhindert oder zumindest deutlich reduziert.

Gegenstand der vorliegenden Erfindung ist in einer ersten Ausführungsform ein Mund- und Zahnpflege- und -reinigungsmittel, enthaltend
a) ein Polyol-Derivat von Arginin der Formel (I) 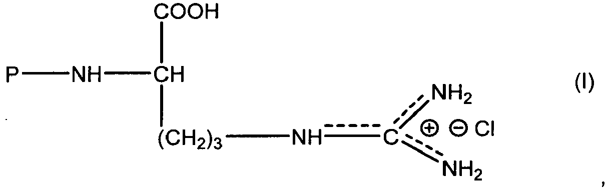 in der P einen Polyol-Rest ausgewählt aus der Gruppe bestehend aus Glycerin, Sorbitol, Hexandiol, Xylitol, Erythritol und Mischungen daraus bedeutet, und
b) 500 bis 1600 ppm Fluorid.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes, Mund- und Zahnspülungen sowie Mund- und Zahngele. Bevorzugt geeignet sind Zahnpasten und flüssige Zahnreinigungsmittel. Hierzu können die Mund- und Zahnpflege- und -reinigungsmittel zum Beispiel in Form von Zahnpasten, flüssigen Zahncremes, Zahnpulvern, Mundwässern oder gegebenenfalls auch als Kaumasse, zum Beispiel als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Eine weitere besonders bevorzugte Ausführungsform der vorliegenden Erfindung sind Mundspüllösungen und Mundwasser, die zum Ausspülen der Mundhöhle verwendet werden.

Als wesentlichen Inhaltsstoff enthalten die Mittel ein Polyol-Derivat von Arginin.

Arginin ist eine proteinogene *α*-Aminosäure mit einer Guanidin-Funktionalität in der Seitenkette. Die Guanidin-Gruppe ist sowohl im sauren und neutralen, als auch im schwach basischen Milieu protoniert und trägt eine positive Ladung, die zwischen den Aminogruppen delokalisiert ist.

In dem Polyol-Derivat ist das Arginin über die freie NH₂-Gruppe mit einem Polyol-Rest verbunden. Der Polyol-Rest leitet sich von Glycerin, Sorbitol, Hexandiol, Xylitol oder Erythritol ab, wobei ein Glycerin-Rest bevorzugt ist.

Die Polyol-Derivate von Arginin können beispielsweise durch Umsetzung der Alkohole, der Aminosäure und Wasserstoff in Gegenwart von Hydrierkatalysatoren erhalten werden.

Die Polyol-Derivate von Arginin werden als Hydrochlorid eingesetzt.

In einer besonders bevorzugten Ausführungsform leitet sich der Rest P von Glycerin ab und das Polyol-Derivat von Arginin weist eine Struktur der Formel (la) auf:

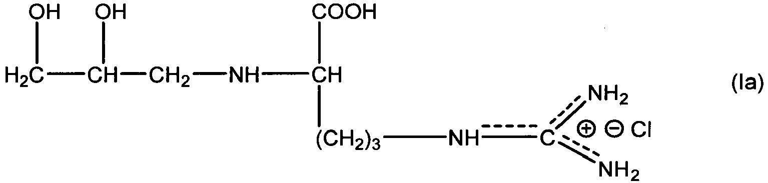

Ein bevorzugtes Polyol-Derivat von Arginin ist beispielsweise unter der INCI-Bezeichnung DIHYDROXYPROPYL ARGININE HCl bekannt. Dieses Polyol-Derivat von Arginin ist unter der Bezeichnung Amitose R von der Firma Seiwa Kasei kommerziell erhältlich.

Bevorzugte Mund- und Zahnpflege- und -reinigungsmittel setzen das Polyol-Derivat von Arginin innerhalb enger Mengenbereiche ein. Hier sind Mund- und Zahnpflege- und -reinigungsmittel bevorzugt, die - bezogen auf ihr Gewicht - 0,01 - 5 Gew.-%, vorzugsweise 0,1 - 4 Gew.-%, besonders bevorzugt 0,5 - 3 Gew.-% und außerordentlich bevorzugt 1 - 2,5 Gew.-% Polyol-Derivat von Arginin enthalten.

Ohne an diese Theorie gebunden sein zu wollen, sind die Polyol-Derivate von Arginin besser in den Mund- und Zahnpflege- und -reinigungsmittel stabilisiert als Arginin und zeigen so auch eine verbesserte Leistung gegen Bakterien und/oder Plaque. Die Polyol-Derivate von Arginin lagern sich in der Plaque ein, werden dort enzymatisch in Arginin und Polyol gespalten und insbesondere das Arginin kann dann vor Ort wirken.

Die Mund- und Zahnpflege- und -reinigungsmittel enthalten weitere Inhaltsstoffe. Bevorzugt ist hierbei der Einsatz von Fluorid. Dieses kann in Form anorganischer Fluoridsalze (Natriumfluorid, Zinn(II)fluorid, Natriummonofluorphosphat usw.) bereitgestellt werden, auch Aminfluoride wie Olaflur sind geeignet.

Es wurde gefunden, dass die Fluoriddeposition durch die Anwesenheit eines Polyol-Derivats von Arginin in den Mitteln oberhalb bestimmter Fluoridgehalte gesteigert werden kann. Die Mindestmenge an Fluorid beträgt dabei 500 ppm, unterhalb dieser Grenze macht sich der Einsatz der Polyol-Derivate von Arginin auf die Fluoriddeposition nicht bemerkbar. Besonders gute Fluoriddepositionswerte werden erzielt, wenn die Polyol-Derivate von Arginin in Gegenwart höherer Fluoriddmengen eingesetzt werden, wobei sich Werte von 1000 ppm Fluorid und darüber als besonders bevorzugt gezeigt haben.

Besonders bevorzugte Mund- und Zahnpflege- und -reinigungsmittel sind dadurch gekennzeichnet, dass sie 1225 bis 1575 ppm, vorzugsweise 1250 bis 1550 ppm, weiter bevorzugt 1275 bis 1525 ppm, noch weiter bevorzugt 1300 bis 1500 ppm, noch weiter bevorzugt 1325 bis 1475 ppm und insbesondere 1350 bis 1450 ppm Fluorid enthalten.

Wird Fluorid in Form von Natriumfluorid bereitgestellt, entspricht 1 Gew.-% Natriumfluorid ungefähr 4524 ppm Fluorid, so dass bevorzugte Mittel 0,27 bis 0,35 Gew.-%, vorzugsweise 0,28 bis 0,34 Gew.-%, weiter bevorzugt 0,29 bis 0,33 Gew.-% und insbesondere 0,30 bis 0,32 Gew.-% Natriumfluorid enthalten.

Als weiteren wichtigen Inhaltsstoff können die Mittel 0,01 bis 10 Gew.-% anionische(s) Tensid(e) enthalten. Besonders bevorzugte Mund- und Zahnpflege- und -reinigungsmittel enthalten 0,5 bis 5 Gew.-%, vorzugsweise 0,75 bis 3 Gew und insbesondere 1 bis 2,5 Gew.-% .-% anionische(s) Tensid(e).

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Ganz besonders bevorzugte Mittel enthalten Alkylsulfat(e) als anionisches Tensid. Hier sind besonders bevorzugte Mund- und Zahnpflege- und -reinigungsmittel dadurch gekennzeichnet, dass sie - bezogen auf ihr Gewicht - 0,5 bis 2,5 Gew.-%, mehr bevorzugt 0,75 bis 2 Gew.-% und insbesondere bevorzugt 1,0 bis 1,5 Gew.-% Natriumlaurylsulfat enthalten.

Ebenfalls ganz besonders bevorzugte Mittel enthalten Fettalkoholethersulfate als anionisches Tensid. Bevorzugte Fettalkoholethersulfate sind solche der Formel

R¹-O-(AO)ₙ-SO₃⁻X⁺.

In dieser Formel steht R¹ für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen.

AO steht für eine Ethylenoxid- (EO) oder Propylenoxid- (PO) Gruppierung, vorzugsweise für eine Ethylenoxidgruppierung. Der Index n steht für eine ganze Zahl von 1 bis 50, vorzugsweise von 1 bis 20 und insbesondere von 2 bis 10. Ganz besonders bevorzugt steht n für die Zahlen 2, 3, 4, 5, 6, 7 oder 8. X steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X+ können ausgewählt sein aus NH₄⁺, ½ Zn²⁺,½ Mg²⁺,½ Ca²⁺,½ Mn²⁺, und deren Mischungen.

Besonders bevorzugte Mund- und Zahnpflege- und -reinigungsmittel enthalten ein Fettalkoholethersulfat ausgewählt aus Fettalkoholethersulfaten der Formel 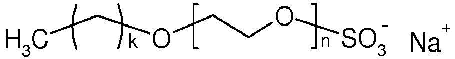 mit k = 11 bis 19, n = 2, 3, 4, 5, 6, 7 oder 8. Ganz besonders bevorzugte Vertreter sind C₁₂₋₁₄ Fettalkoholethersulfate mit 2 EO (k = 11-13, n = 2) und dabei insbesondere deren Na-Salze. Der angegebenen Ethoxylierungsgrad stellt einen statistischen Mittelwert dar, der für ein spezielles Produkt eine ganze oder eine gebrochene Zahl sein kann.

Hier sind besonders bevorzugte Mund- und Zahnpflege- und -reinigungsmittel dadurch gekennzeichnet, dass sie 0,5 bis 2,5 Gew.-%, mehr bevorzugt 0,75 bis 2 Gew.-% und insbesondere bevorzugt 1,0 bis 1,5 Gew.-% Natriumlaurylethersulfat mit 2 EO enthalten.

Besonders bevorzugt ist auch der Einsatz von sogenannten Feuchthaltemitteln, die bei Zahnpasten das Austrocknen verhindern und die Wirksamkeit der Polyol-Derivate von Arginin steigern. Bei sogenannten Flüssigzahncremes mit fließfähiger Rheologie dienen diese Feuchthaltemittel als Matrix und werden in höheren Mengen eingesetzt. Bei Mundwässern und Mundspülungen dienen diese Feuchthaltemittel als Konsistenzregler und zusätzliche Süßungsmittel. Geeignete Feuchthaltemittel umfassen insbesondere einen mehrwertigen Alkohol aus der Gruppe Sorbit und/oder Glycerin und/oder 1,2-Propylenglycol.-%.

Hier sind Mund- und Zahnpflege- und -reinigungsmittel bevorzugt, die - bezogen auf ihr Gewicht - 0,5 bis 70 Gew.-%, vorzugsweise 0,75 bis 60 Gew.-%, besonders bevorzugt 1 bis 50 Gew.-% und insbesondere 2 bis 40 Gew.-% mindestens eines mehrwertigen Alkohols aus der Gruppe bestehend aus Sorbit, Glycerin, 1,2-Propylenglycol.-% und Mischungen daraus enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können zum Beispiel Xylit, Propylenglycole, Polyethylenglycole, insbesondere solche mit mittleren Molekulargewichten von 200-800 eingesetzt werden.

Als weiteren Inhaltsstoff können die Mittel weitere Tensid(e), insbesondere amphotere Tenside, enthalten. Auch die weiteren Tenside werden vorzugsweise innerhalb enger Mengenbereiche eingesetzt, so dass bevorzugte Mund- und Zahnpflege- und -reinigungsmittel dadurch gekennzeichnet sind, dass sie 0,5 bis 5 Gew.-%, vorzugsweise 0,75 bis 4,5 Gew.-%, weiter bevorzugt 1 bis 4 Gew.-%, noch weiter bevorzugt 1,25 bis 3,5 Gew.-% und insbesondere 1,6 bis 2,5 Gew.-% Tensid(e) enthalten.

Besonders bevorzugte Mund- und Zahnpflege- und -reinigungsmittel enthalten amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyldimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen.

Besonders bevorzugte Mund- und Zahnpflege- und -reinigungsmittel enthalten als amphotere Tenside Betaine der Formel (Bet-I) 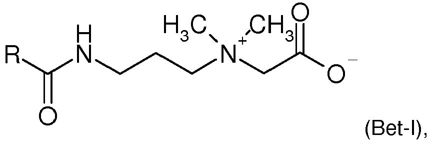 in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden Tenside der Formel (Bet-I) eingesetzt, die ein Gemisch der folgenden Vertreter sind:

H₃C-(CH₂)₇-C(O)-NH-(CH₂)₃N⁺(CH₃)₂CH₂COO⁻

H₃C-(CH₂)₉-C(O)-NH-(CH₂)₃N⁺(CH₃)₂CH₂COO⁻

H₃C-(CH₂)₁₁-C(O)-NH-(CH₂)₃N⁺(CH₃)₂CH₂COO⁻

H₃C-(CH₂)₁₃-C(O)-NH-(CH₂)₃N⁺(CH₃)₂CH₂COO⁻

H₃C-(CH₂)₁₅-C(O)-NH-(CH₂)₃N⁺(CH₃)₂CH₂COO⁻

H₃C-(CH₂)₇-CH=CH-(CH₂)₇-C(O)-NH-(CH₂)₃N⁺(CH₃)₂CH₂COO⁻

Besonders bevorzugt werden Tenside der Formel (Bet-I) innerhalb enger Mengenbereiche eingesetzt. Hier sind Mund- und Zahnpflege- und -reinigungsmittel bevorzugt, die - bezogen auf ihr Gewicht - 0,1 bis 4,0 Gew.-%, vorzugsweise 0,2 bis 3,0 Gew.-%, besonders bevorzugt 0,3 bis 2,5 Gew.-%, weiter bevorzugt 0,4 bis 2,0 Gew.-% und insbesondere 0,5 bis 1,6 Gew.-% Cocamidopropylbetain enthalten.

Zur Entfaltung der Reinigungsleistung und für eine "natürliche" Farbaufhellung von Zähnen können insbesondere die Zahnpflege- und -reinigungsmittel Poliermittel enthalten. Als Poliermittel eignen sich prinzipiell alle für Zahnpflege- und -reinigungsmittel bekannten Putzkörper. Bevorzugt geeignete Poliermittel-komponenten sind Kieselsäuren, Aluminiumhydroxid, Aluminiumoxid, Natriumaluminiumsilikate, organische Polymere, Calciumcarbonat oder Gemische solcher Putzkörper.

Calciumhaltige Polierkomponenten wie zum Beispiel Kreide, synthetisches Calciumcarbonat, Calciumpyrophosphat, Dicalciumphosphatdihydrat können in Mengen bis zu 5 Gew.-% - bezogen auf das gesamte Mittel - enthalten sein.

Der Gesamtgehalt an Poliermitteln liegt vorzugsweise im Bereich von 5 - 50 Gew.-% des Zahnpflege- und -reinigungsmittels.

Besonders bevorzugt sind Zahnpasten und flüssige Zahnreinigungsmittel, die als Poliermittel Kieselsäuren enthalten. Geeignete Kieselsäuren sind zum Beispiel Gelkieselsäuren, Hydrogelkieselsäuren und Fällungskieselsäuren. Gelkieselsäuren werden durch Umsetzung von Natriumsilikatlösungen mit starken, wässrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sogenannten Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident®12 DS (Evonik) erhältlich.

Andere Fällungskieselsäuren dieser Art sind Sident® 8 (Evonik) und Sorbosil® AC 39 (PQ Corp.). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungskieselsäuren mit einer BET-Oberfläche von 150-250 m²/g zu, zum Beispiel die Handelsprodukte Sipernat® 22 LS oder Sipernat® 320 DS.

Ebenso kann als Poliermittel auch Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich. Es ist jedoch bevorzugt, dass Aluminiumoxid als weiteres Poliermittel, das heißt in Kombination mit einem Poliermittel, in den Mitteln eingesetzt wird.

Als Poliermittel eignen sich weiter alle für Zahnpasten bekannten Putzkörper wie zum Beispiel Natriumaluminiumsilikate wie zum Beispiel Zeolith A, organische Polymere wie zum Beispiel Polymethacrylat oder Gemische dieser und der vorstehend genannten Putzkörper.

Zusammenfassend sind Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, synthetisches Calciumcarbonat, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Putzkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Putzkörpern, wobei einzelne Putzkörper vorzugsweise in engeren Mengenbereichen eingesetzt werden. Bevorzugte Mittel enthalten beispielsweise 5 bis 20 Gew.-%, vorzugsweise 8 bis 21 Gew.-%, weiter bevorzugt 9 bis 20 Gew.-% und insbesondere 10 bis 19 Gew.-% Kieselsäure(n). Weiter bevorzugte Mittel sind dadurch gekennzeichnet, dass sie 0,25 bis 2 Gew.-%, vorzugsweise 0,5 bis 1,5 Gew.-% und insbesondere 0,75 bis 1,25 Gew.-% Aluminiumoxid als weiteres Poliermittel zu der/den Kieselsäure(n) enthalten.

Es kann auch bevorzugt sein, natürliches und/oder synthetisches Calciumcarbonat, insbesondere in Kombination mit Fluorid, als Poliermittel in den Mund- und Zahnpflege- und -reinigungsmittel einzusetzen.

Als Konsistenzregler (bzw. Bindemittel) dienen zum Beispiel natürliche und/oder synthetische wasserlösliche Polymere wie Alginate, Carragheenate, Traganth, Stärke und Stärkeether, Celluloseether wie zum Beispiel Carboxymethylcellulose (Na-Salz), Hydroxyethylcellulose, Methylhydroxypropylcellulose, Guar, Akaziengum, Agar-Agar, Xanthan-Gum, Succinoglycan-Gum, Johannisbrotmehl, Pektine, wasserlösliche Carboxyvinylpolymere (zum Beispiel Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind zum Beispiel Schichtsilikate wie zum Beispiel Montmorillonit-Tone, kolloidale Verdickungskieselsäuren wie zum Beispiel Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol-Copolymere mit einem mittleren Molgewicht von 1000 bis 5000 oder eine Kombination der genannten Verbindungen in den Zahnpasten verwendet werden.

Als besonders verträglich mit den weiteren Wirkstoffen der Mund- und Zahnpflege- und - reinigungsmittel haben sich CMC und Xanthan erwiesen. Bei Einsatz dieser Verdickern ist die Wirkung besonders ausgeprägt. Besonders bevorzugte Mund- und Zahnpflege- und - reinigungsmittel sind demnach dadurch gekennzeichnet, dass sie - bezogen auf ihr Gewicht - 0,2 bis 7,5 Gew.-%, vorzugsweise 0,25 bis 5 Gew.-%, besonders bevorzugt 0,3 bis 4 Gew.-%, weiter bevorzugt 0,4 bis 3 Gew.-%, noch weiter bevorzugt 0,45 bis 2 Gew.-% und insbesondere 0,5 bis 0,8 Gew.-% Carboxymethylcellulose enthalten.

Weitere besonders bevorzugte Mund- und Zahnpflege- und -reinigungsmittel sind dadurch gekennzeichnet, dass sie - bezogen auf ihr Gewicht - 0,15 bis 5 Gew.-%, vorzugsweise 0,2 bis 2,5 Gew.-%, besonders bevorzugt 0,25 bis 1 Gew.-%, weiter bevorzugt 0,3 bis 0,75 Gew.-%, noch weiter bevorzugt 0,35 bis 0,6 Gew.-% und insbesondere 0,4 bis 0,5 Gew.-% Xanthan enthalten.

Die Mittel, insbesondere die Zahnpflege- und -reinigungsmittel, können auch Substanzen zur Erhöhung des mineralisierenden Potentials enthalten, beispielsweise calciumhaltige Substanzen wie zum Beispiel Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflege- und -reinigungsmittel enthaltenen Substanzen ab. Neben den genannten obligatorischen Komponenten können die Mund- und Zahnpflege- und - reinigungsmittel weitere, an sich bekannte Hilfs- und Zusatzstoffe enthalten. Dabei ist ein Zusatzstoff, der als Zahnpastenkomponente seit langem bekannt ist, in den Mund- und Zahnpflege- und - reinigungsmittel besonders wirksam: Calciumglycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den Mund- und Zahnpflege- und -reinigungsmittel wird Calciumglycerophosphat bevorzugt in Mengen von 0,01 - 1 Gew.-% eingesetzt. Insgesamt können die Mund- und Zahnpflege- und -reinigungsmittel übliche Hilfsmittel und Zusatzstoffe in Mengen bis zu 10 Gew.-% enthalten.

Die Mund- und Zahnpflege- und -reinigungsmittel können zum Beispiel durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind zum Beispiel Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind zum Beispiel Saccharin-Natrium, Natrium-Cyclamat, Saccharose, Lactose, Maltose, Fructose.

Weitere übliche Hilfs- und Zusatzstoffe für Zahnpasten oder Zahncremes und Mundwässer oder Mundspüllösungen sind
- Lösungsmittel und Lösungsvermittler, z.B. niedere einwertige oder mehrwertige Alkohole oder Ether, z.B. Ethanol, 1,2-Propylenglycol, Diethylenglycol oder Butyldiglycol
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid,
- Vitamine wie z.B. Ascorbinsäure, Biotin oder Tocopherol,
- Mineralsalze wie z.B. Mangan-, Zink- oder Magnesiumsalze oder Natriumphosphate.

Weitere Gegenstände der Erfindung sind ein Polyol-Derivat von Arginin der Formel (I)

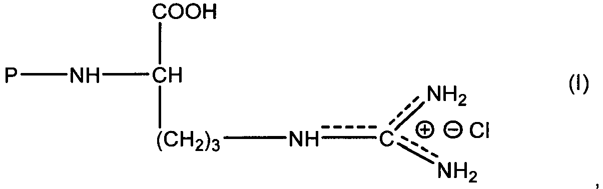

in der P einen Polyol-Rest ausgewählt aus der Gruppe bestehend aus Glycerin, Sorbitol, Hexandiol, Xylitol, Erythritol und Mischungen daraus bedeutet, sowie ein Mund- und Zahnpflege- und -reinigungsmittel enthaltend a) ein Polyol-Derivat von Arginin der Formel (I) 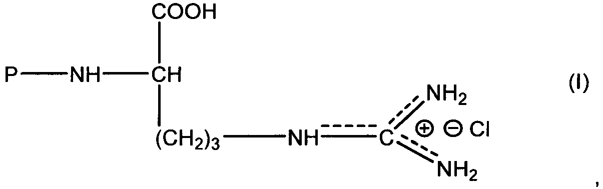 in der P einen Polyol-Rest ausgewählt aus der Gruppe bestehend aus Glycerin, Sorbitol, Hexandiol, Xylitol, Erythritol und Mischungen daraus bedeutet, und b) 500 bis 1600 ppm Fluorid, zur Verwendung bei der Lösung und/oder Entfernung von extrazellulären polymeren Substanzen (EPS) auf Zahnoberflächen und/oder von Zahnbelag (Plaque) und/oder von Zahnstein und/oder bei der Reduktion und/oder Vermeidung einer pH-Absenkung an der Zahnoberfläche und/oder bei der Remineralisierung von Zahnschmelz und/oder zur Vermeidung einer Entmineralisierung von Zahnschmelz.

Bezüglich bevorzugter Ausführungsformen der Verwendungen gilt mutatis mutandis das zu den Mitteln Gesagte.

Die Mittel können als Zahnpasten oder Zahncremes formuliert werden.

Die folgenden Beispiele sollen den Gegenstand der vorliegenden Erfindung erläutern ohne ihn jedoch zu beschränken.

### Beispiele:

Alle Angaben in Gew.-%.

**Tabelle 1: Zahnpastaformulierungen**

| | **A** | **B** | **C** | **D** | **E** | **F** |
|---|---|---|---|---|---|---|
| Sorbitol, 70%ig | 55 | 55 | 45 | 60 | 65 | 70 |
| Dihydroxypropyl Arginine HCl | 1 | 2,5 | 0,5 | 1 | 1 | 1,5 |
| Hydrated silica | 12 | 20 | 10 | 5 | 12,5 | 14 |
| Natriumfluorid | 0,32 | 0,32 | 0,32 | 0,32 | 0,32 | 0,1 |
| Natrium Saccharin | 0,25 | 0,25 | 0,1 | 0,5 | 0,2 | 0,25 |
| Ethanol | -- | -- | -- | 1 | 2 | -- |
| Xanthan | 0,6 | 0,6 | 0,3 | 0,2 | 0,2 | 0,1 |
| Natriumlaurylsulfat | 1,5 | 0,5 | 1,5 | 1,2 | 0,8 | 1,2 |
| Cocamidopropyl Betaine | 1,3 | 1,3 | 0,6 | 0,6 | 0,6 | 0,6 |
| PEG-8 | 1,5 | 1,0 | 2,5 | 4,0 | 1,0 | 3,0 |
| Na₂HPO₄ | 0,2 | 0,2 | 0,2 | 0,2 | 0,5 | 0,2 |
| Aroma | 1,1 | 1,1 | 0,75 | 1,5 | 0,5 | 0,75 |
| Wasser, Farbstoff und ggf. Konservierung | ad 100 | | | | | |

**Tabelle 2: Mundwasserformulierung**

| | **A** | **B** | **C** | **D** | **E** | **F** |
|---|---|---|---|---|---|---|
| Sorbitol, 70%ig | 1 | 1,5 | 2 | 2,5 | 3 | 5 |
| Dihydroxypropyl Arginine HCl | 0,75 | 3,0 | 2,5 | 5 | 0,5 | 1 |
| Hydrated silica | 1 | 1,5 | 2 | 2,5 | 1 | 1 |
| Natriumfluorid | 0,11 | 0,11 | 0,11 | 0,11 | 0,11 | 0,11 |
| Natrium Saccharin | 0,03 | 0,05 | 0,1 | 0,05 | 0,05 | 0,1 |
| PEG-60 Hydrogenated Castor Oil | 0,2 | 0,2 | 0,2 | 0,2 | 0,2 | 0,2 |
| Trinatriumcitrat Dihydrat | 0,1 | 0,2 | 0,3 | 0,2 | 0,1 | 0,1 |
| Citronensäure | 0,001 | 0,002 | 0,1 | 0,2 | 0,01 | 0,01 |
| Cetylpyridinium Chloride | 0,01 | 0,1 | 0,05 | 0,05 | 0,1 | 0,01 |
| Aroma | 0,5 | 0,5 | 0,5 | 0,5 | 0,5 | 0,5 |
| Wasser | ad 100 | | | | | |

## Patentansprüche

1. Mund- und Zahnpflege- und -reinigungsmittel, enthaltend
a) ein Polyol-Derivat von Arginin der Formel (I) 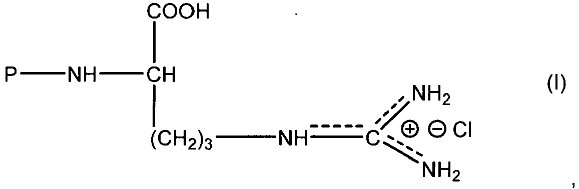 in der P einen Polyol-Rest ausgewählt aus der Gruppe bestehend aus Glycerin, Sorbitol, Hexandiol, Xylitol, Erythritol und Mischungen daraus bedeutet, und
b) 500 bis 1600 ppm Fluorid.

2. Mund- und Zahnpflege- und -reinigungsmittel nach Anspruch 1, **dadurch gekennzeichnet, dass** es - bezogen auf sein Gewicht - 0,01 - 5 Gew.-%, vorzugsweise 0,1 - 4 Gew.-%, besonders bevorzugt 0,5 - 3 Gew.-% und außerordentlich bevorzugt 1 - 2,5 Gew.-% Polyol-Derivat von Arginin der Formel (I) enthält.

3. Mund- und Zahnpflege- und -reinigungsmittel nach einem der Ansprüche 1 oder 2, **dadurch gekennzeichnet, dass** das Polyol-Derivat von Arginin eine Struktur der Formel (la) aufweist

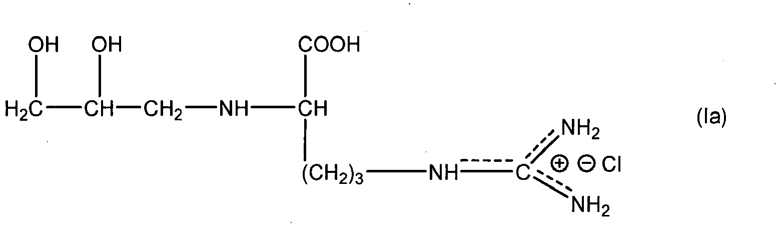

4. Mund- und Zahnpflege- und -reinigungsmittel nach einem der Ansprüche 1 bis 3, **dadurch gekennzeichnet, dass** das Mund- und Zahnpflege- und -reinigungsmittel weiterhin Calciumcarbonat enthält.

5. Polyol-Derivats von Arginin der Formel (I) 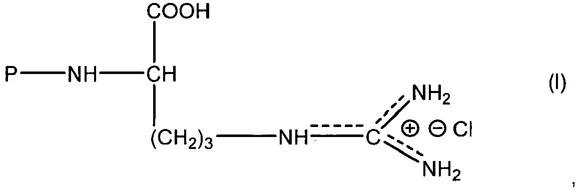 in der P einen Polyol-Rest ausgewählt aus der Gruppe bestehend aus Glycerin, Sorbitol, Hexandiol, Xylitol, Erythritol und Mischungen daraus bedeutet, zur Verwendung bei der Lösung und/oder Entfernung von extrazellulären polymeren Substanzen (EPS) auf Zahnoberflächen und/oder von Zahnbelag (Plaque) und/oder von Zahnstein und/oder bei der Reduktion und/oder Vermeidung einer pH-Absenkung an der Zahnoberfläche und/oder bei der Remineralisierung von Zahnschmelz und/oder bei der Vermeidung einer Entmineralisierung von Zahnschmelz.

6. Mund- und Zahnpflege- und -reinigungsmittel enthaltend
a) ein Polyol-Derivat von Arginin der Formel (I) 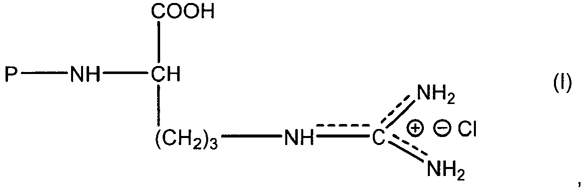 in der P einen Polyol-Rest ausgewählt aus der Gruppe bestehend aus Glycerin, Sorbitol, Hexandiol, Xylitol, Erythritol und Mischungen daraus bedeutet, und
b) 500 bis 1600 ppm Fluorid
zur Verwendung bei der Lösung und/oder Entfernung von extrazellulären polymeren Substanzen (EPS) auf Zahnoberflächen und/oder von Zahnbelag (Plaque) und/oder von Zahnstein und/oder bei der Reduktion und/oder Vermeidung einer pH-Absenkung an der Zahnoberfläche und/oder bei der Remineralisierung von Zahnschmelz und/oder bei der Vermeidung einer Entmineralisierung von Zahnschmelz.

## Claims

1. An oral and dental care and cleaning agent, containing
a) a polyol derivative of arginine of formula (I) 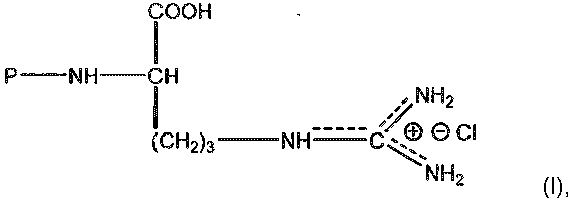 in which P denotes a polyol group selected from the group consisting of glycerol, sorbitol, hexanediol, xylitol, erythritol and mixtures thereof, and
b) 500 to 1600 ppm fluoride.

2. The oral and dental care and cleaning agent according to claim 1, **characterized in that** it contains, based on its weight, 0.01-5 wt.%, preferably 0.1-4 wt.%, particularly preferably 0.5-3 wt.%, and extremely preferably 1-2.5 wt.% polyol derivative of arginine of formula (I).

3. The oral and dental care and cleaning agent according to one of claims 1 or 2, **characterized in that** the polyol derivative of arginine has a structure of formula (Ia)

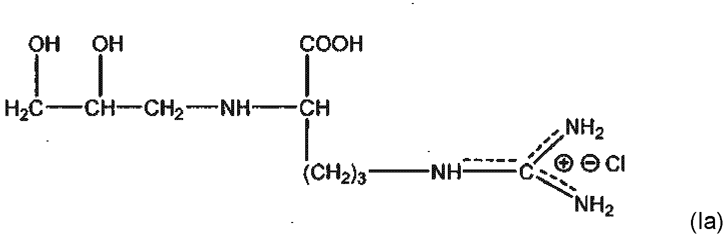

4. The oral and dental care and cleaning agent according to one of claims 1 to 3, **characterized in that** the oral and dental care and cleaning agent further contains calcium carbonate.

5. A polyol derivative of arginine of formula (I) 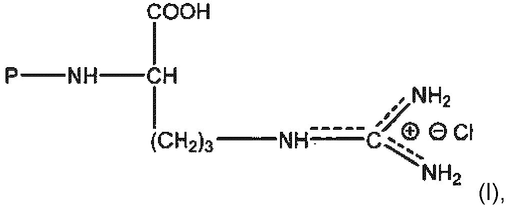 in which P denotes a polyol group selected from the group consisting of glycerol, sorbitol, hexanediol, xylitol, erythritol and mixtures thereof, for use in dissolving and/or removing extracellular polymeric substances (EPS) on tooth surfaces and/or plaque and/or tartar and/or in the reduction and/or avoidance of a pH drop on the tooth surface and/or in the remineralization of tooth enamel and/or in the avoidance of demineralization of tooth enamel.

6. An oral and dental care and cleaning agent, containing
a) a polyol derivative of arginine of formula (I) 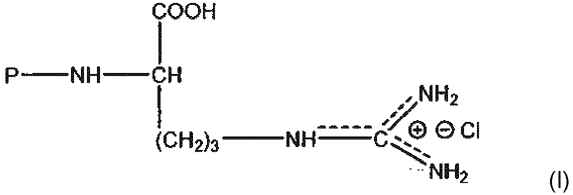 in which P denotes a polyol group selected from the group consisting of glycerol, sorbitol, hexanediol, xylitol, erythritol and mixtures thereof, and
b) 500 to 1600 ppm fluoride
for use in dissolving and/or removing extracellular polymeric substances (EPS) on tooth surfaces and/or plaque and/or tartar and/or in the reduction and/or avoidance of a pH drop on the tooth surface and/or in the remineralization of tooth enamel and/or in the avoidance of demineralization of tooth enamel.

## Revendications

1. Produit de soin et d'hygiène bucco-dentaire contenant
a) un dérivé polyol d'arginine de formule (I) 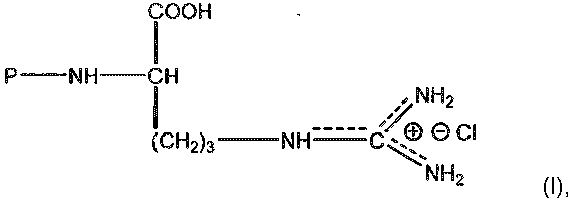 où P désigne un radical polyol choisi dans le groupe constitué par le glycérol, le sorbitol, l'hexanediol, le xylitol, l'érythritol et leurs mélanges, et
b) 500 à 1600 ppm de fluorure.

2. Produit de soin et d'hygiène bucco-dentaire selon la revendication 1, **caractérisé en ce qu'**il contient - sur la base de son poids - 0,01 à 5 % en poids, de préférence 0,1 à 4 % en poids, de manière particulièrement préférée 0,5 à 3 % en poids et de manière extrêmement préférée 1 à 2,5 % en poids, d'un dérivé polyol d'arginine de formule (I).

3. Produit de soin et d'hygiène bucco-dentaire selon l'une des revendications 1 ou 2, **caractérisé en ce que** le dérivé polyol d'arginine présente une structure de formule (la)

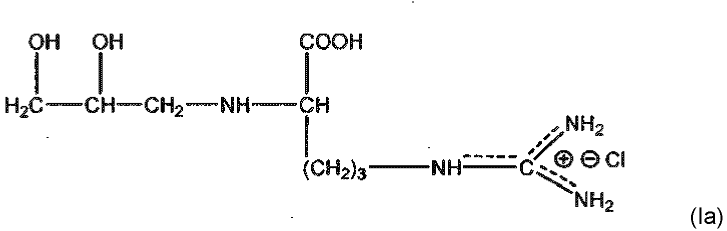

4. Produit de soin et d'hygiène bucco-dentaire selon l'une des revendications 1 à 3, **caractérisé en ce que** le produit de soin et d'hygiène bucco-dentaire contient en outre du carbonate de calcium.

5. Dérivé polyol d'arginine de formule (I) 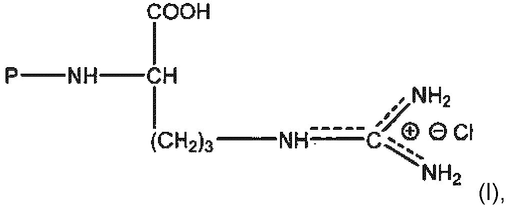 où P désigne un radical polyol choisi dans le groupe constitué par le glycérol, le sorbitol, l'hexanediol, le xylitol, l'érythritol et leurs mélanges, destiné à être utilisé pour dissoudre et/ou éliminer des substances polymères extracellulaires (EPS) sur des surfaces dentaires et/ou de la plaque dentaire et/ou du tartre dentaire, et/ou pour réduire et/ou éviter une baisse de pH sur la surface dentaire, et/ou pour reminéraliser l'émail dentaire et/ou pour éviter une déminéralisation de l'émail dentaire.

6. Produit de soin et d'hygiène bucco-dentaire contenant
a) un dérivé polyol d'arginine de formule (I) 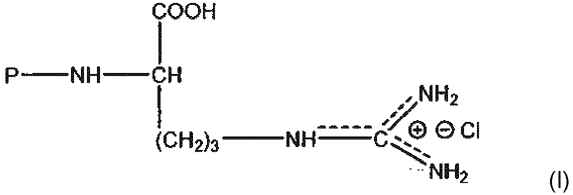 où P désigne un radical polyol choisi dans le groupe constitué par le glycérol, le sorbitol, l'hexanediol, le xylitol, l'érythritol et leurs mélanges, et
b) 500 à 1600 ppm de fluorure
destiné à être utilisé pour dissoudre et/ou éliminer des substances polymères extracellulaires (EPS) sur des surfaces dentaires et/ou de la plaque dentaire et/ou du tartre dentaire, et/ou pour réduire et/ou éviter une baisse de pH sur la surface dentaire, et/ou pour reminéraliser l'émail dentaire et/ou pour éviter une déminéralisation de l'émail dentaire.
